# EUROPEAN PATENT SPECIFICATION

(11) **EP 3 113 804 B1**
(45) Date of publication and mention of the grant of the patent: **25.12.2019**
(21) Application number: 15708495.5
(22) Date of filing: 05.03.2015
(51) Int. Cl.: A61L 27/06, A61C 8/00, A61L 27/56

(54) **IMPLANT SURFACE COMPOSITION**
IMPLANTATOBERFLÄCHENZUSAMMENSETZUNG
COMPOSITION DE SURFACE D'IMPLANT

(30) Priority: 07.03.2014 GB 201404011
(43) Date of publication of application: 11.01.2017
(73) Proprietor: Nobel Biocare Services AG, 8302 Kloten (CH)
(72) Inventor: HALL, Jan, S-416 80 Göteborg (SE)
(74) Representative: Capré, Didier
(86) International application number: PCT/EP2015/054573
(87) International publication number: WO 2015/132325

(56) References cited:
- WO-A1-2005/055860
- CN-A- 101 307 479
- US-B1- 7 708 558
- JAEGGI C ET AL: "Anodic thin films on titanium used as masks for surface micropatterning of biomedical devices", SURFACE AND COATINGS TECHNOLOGY, ELSEVIER, AMSTERDAM, NL, vol. 200, no. 5-6, 21 November 2005 (2005-11-21), pages 1913-1919, XP027608760, ISSN: 0257-8972 [retrieved on 2005-11-21]
- CHEN Z X ET AL: "Surface characteristics and in vitro biocompatibility of titanium anodized in a phosphoric acid solution at different voltages", BIOMEDICAL MATERIALS, INSTITUTE OF PHYSICS PUBLISHING, BRISTOL, GB, vol. 4, no. 6, 1 December 2009 (2009-12-01), page 65003, XP020170651, ISSN: 1748-605X

## Description

### Field of the Invention

The invention pertains in general to the field of an implant structure or parts thereof having a surface composition suitable for an implant system in particular a dental implant system. The invention also relates to a method for production of an implant structure and an implant system and parts or portions thereof provided with an implant structure .

### Background of the Invention

For the past 40 years dental implants have been successfully used to replace lost or missing teeth. One of the overriding challenges in implant dentistry is to modify the properties of soft tissue adhering surfaces to promote optimal soft-tissue adherence at the same time as minimizing bacterial adhesion and bio film formation and allowing the maintenance of good oral hygiene. In order to minimize the risk of microbial colonization on e.g. the spacer surface, the coronal part of the implant or any device in contact with soft tissue, it is believed that the surfaces shall be smooth with structures that limit bacteria growth. Therefore, it is common to manufacture the coronal implant system parts by machining, such as milling and/or turning, which results in smooth surfaces with structures in the sub- or low micrometer range. Implants and spacer sleeves with such surfaces have been used successfully for decades.

In most cases, soft tissue integration is good and bacteria growth is limited on the machined structures and peri-implant infections can be avoided. However, they do occur, and a few percent of all patients rehabilitated with dental implants experience complications due to peri-implant infections. It is therefore desired to continue to reduce the risk of peri-implant infection while maintaining good soft tissue integration. Peri-implantitis is an inflammatory process that affects the soft and hard tissues surrounding an osseointegrated implant, and this process is accompanied by specific pathogenic microbes which are identified in the peri-implant pockets. The aethiology of peri-implantitis is multifactorial and up to now not really well understood. It is influenced by the microbial composition in the oral cavity, the genetic disposition and/or immune status of the patient, the practiced oral hygiene, and the physical condition and age of the patient. Microbial plaque accumulation and severe bacterial infection may lead as a consequence to rapid bone loss associated with bleeding and suppuration.

The end point of perimplantitis can be implant failure (Roos-Jansaker et al. 2006; Fransson et al. 2009; Koldsland et al. 2010). This condition has been described as the breakdown of bone tissue as a response to inflammation resulting from bacterial infection surrounding an endosseous dental implant (Lindhe & Meyle, 2008). On placement of an implant into the oral cavity exposed surfaces of a spacer and in some cases also parts of the neck of the implant, which emerge through the mucosa, immediately become covered with an acquired pellicle of proteins derived from the surrounding environment. Studies have shown the predominant salivary proteins that adhere to titanium surfaces *in vitro* to include proline-rich proteins, high-molecular weight mucin, secretory IgA, zinc-α₂-glycoprotein, cystatins, α-amylase and prolactin-induced protein (Edgerton et al. 1996; Lima et al. 2008; Dorkhan et al. 2013). Adsorbed proteins provide a range of binding sites for oral bacteria to attach and initiate the development of a biofilm. Early colonizers of oral surfaces include Streptococci (*Streptococcus oralis, Stretococcus mitis, Streptocoocus gordonii* and *Streptococcus sanguinis*) as well as species such as *Actinomyces naeslundii* (Nyvad & Killian, 1987; Kolenbrander et al. 2002). Streptococci are known to express surface adhesins that bind to salivary proteins (Nobbs et al. 2009). These include the Agl/ll proteins which mediate interactions with, for example, salivary agglutinin (gp340), proline-rich proteins and salivary glycoproteins (Jenkinson & Lamont, 1997).

Once colonization has been initiated, the primary colonizers present new sites for adhesion of secondary colonizers and biofilm formation are thereby initiated (Kolenbrander et al. 2002). Continuous undisturbed growth of such biofilms can result in gradual colonization of the whole spacer surface including the most coronal implant area. In some cases, extensive biofilm growth may result in an infection and an inflammatory host response that result in tissue breakdown. Such a condition is usually referred to as peri-implantitis, and it may lead to implant failure. One of the factors thought to be important in preventing peri-implant infections is the formation of a tight soft-tissue seal around the neck of the implant (Lindhe & Berglundh, 1998).

It is known that anaerobic bacterium may reside inside of placed dental implants. In some cases a gap between the crown/abutment connected to the implant may be present and it may establish a supply of nutrients from the saliva into the inside cavity of the implant, but also cause microbial leakage in that it enables bacteria to move in and out of the cavity in unfortunate conditions. Bacteria may affect the corrosion behavior of metal and alloys immersed in an aqueous environment.

Titanium, which may be used as a dental implant, is generally corrosion resistant, but it is not inert to corrosive attacks, especially when the oxide layer is thin. Titanium is known to have a natural oxide layer having a thickness ranging from 3 nm to 7 nm on the machined surfaces of the material. Although this layer gives some protection to the metal, a continuous attack of weak acids can induce the leaking of metal ions that are transported through the gap into the surrounding tissues. The liberation of elements from the metal or alloys can lead to product changes such as roughening of the surface and weakening of the strength of the construction material, but it might also induce toxic reactions. It is known that free titanium ions inhibit growth of hydroxyapatite crystals and thereby hinder the mineralization of calcified tissue

Various implant structures of fixtures, spacers and prosthetic components have been presented over the years. In addition, various substances and compositions have been proposed for being used as coatings on substrates, such as to form an implant surface. However, as will be recognized after having read the technical background of the present application the human body is a highly advanced and complex environment and it is not an obvious task how to design an implant structure to overcome the described issues. Bearing in mind the complexity of the environment it is also an object of the present invention to bring forward a solution that improves the resistance against peri-implantitis without compromising on the benefits of established solutions.

One way to treat implants that have been used for and shown excellent result for osseointegration in bone is the TiUnite® surface. The surface treatment is obtained by means of so-called anodic oxidation, based on known methods according to Swedish patents 99019474-7 and 0001202-1. However, this oxidation method was not proposed to function in the crystalline range in the said patents. Applying the method to function in the crystalline range is proposed in application WO2005055860. However, the Swedish patents 99019474-7, 0001202-1 as well as WO2005055860 disclose a method used in order to obtain a porous and roughened surface. Reference is also made to JP 2000116673 and JP 11033106, Kokubo et al. relating to e.g a bone implant material which can be used in the crystalline range.
There is a continuing desire to develop dental implants that prevent peri-implantitis. There is also a strong demand for a high predictability of success and long lasting survival rates of dental implants. In addition, it would be advantageous to create an implant that is aesthetically pleasing, and can promote gingival health.
Further related art may be found in JAEGGI C et al., Surface and Coating Technology 200 (2005) 1913-1919 titled "Anodic thin films on titanium used as masks for surface micro patterning of biomedical devices*".*

### Summary of the Invention

Accordingly, embodiments of the present invention preferably seek to mitigate, alleviate or eliminate one or more deficiencies, disadvantages or issues in the art, such as the above-identified, singly or in any combination by providing a medical implant and a method of implanting a medical implant according to the appended patent claims.

The following description focuses on an embodiment of the present invention applicable to implants and spacer sleeves, or units passing through soft tissue, and methods for production of implants and such units in the dental field are already well known on the market and from descriptions in the patent literature and the general literature. Most of the known implants and units are designed in line with a general attempt to achieve good implantation results at reasonable costs. There is therefore a general need to obtain, between the implant and the jaw bone and between the part of the implant and/or unit extending through the soft tissue and the soft tissue, a good and also esthetical satisfactory integration which does not tend to problems for the patient.

The present invention is directed to an implant structure or parts thereof having a surface composition obtainable through an anodic oxidation process, wherein said surface composition comprising titanium oxide in the anatase crystalline phase and that said surface composition has a porous structure where at least 90% of the pores have an orifice with a mean inside diagonal distance of less than 0,1 µm.

According to an embodiment of the present invention an implant structure or parts thereof having a surface composition, wherein said surface composition comprising titanium oxide in the anatase crystalline phase and that said surface composition has a porous structure where at least 90% of the pores have an orifice with a mean inside diagonal distance of less than 0,1 µm. Hence, it has been found and contrary to what was known before that titanium dioxide can be arranged in a crystalline phase on the surfaces of the implant and spacer sleeves while preserving the structure in the micrometer range resulting from manufacturing the components by machining. It will be demonstrated below that the crystalline phase inhibits bacteria colonization, and thus, the properties for limited bacteria growth obtained by the smooth machined surface is further enhanced by adding crystalline titanium oxide in the anatase phase to the surface.

More preferably, the surface composition has a mean roughness value (Sa value) of below 0,3 µm. This is especially useful in soft tissue facing portions.

Preferably, at least 95% of the pores, preferably at least 99% of the pores have an orifice with a mean inside diagonal distance of less than 0,2 µm.

According to a preferred embodiment of the invention the surface composition forms part of a tissue facing surface of a component of an implant system. As will be understood it is a special objective that the surface composition forms part of a soft tissue facing surface of a component of an implant system.

According to a second aspect of the invention the surface composition forms part of a bore or other inner surface of a component of an implant system and more preferably a dental implant system. As will be realized the effect of microbial corrosion may be reduced by utilizing the teachings of the invention.

More in detail at least 35% by volume of said surface composition is formed by titanium oxide in the anatase phase. Preferably, a major content, i.e. more than half, of said surface composition volume is formed by titanium oxide in the anatase phase. In addition the surface composition may comprise phosphorus.

In accordance with yet another alternative embodiment of the invention a coating is provided on said surface composition, preferably said coating comprises an antimicrobial agent.

More preferred an implant system is provided with at least one component having an implant structure according to the present invention. Said implant system is advantageously a dental implant system comprising a body having a proximal end, a distal end, an outer surface extending between the proximal end and the distal end, the body having a longitudinal axis; and an open socket formed in a top portion of the body, the open socket comprising an inner surface extending from the distal end towards the proximal end of the body along the longitudinal axis of the body; and a spacer, e. g. a spacer sleeve, belonging to said dental implant system, which is/are intended to extend from said distal end in a hole formed in jaw bone and in soft tissue.

The spacer is meant to be chosen from the group comprising abutments, overdenture bars and fixture supported bridges. The components are well known to the skilled person and known to have a soft tissue facing part and will not need to be explained or shown in any detail here or in the following. Throughout the whole application it is understood that spacer is a more generic term than abutment. However, an abutment for use in the oral cavity is also often referred to as a spacer so in any future applications derived from the present application the word abutment may be used instead of spacer to more clearly define the scope of protection around one preferred embodiment, which is a fixture supported dental abutment.

A dental implant system is provided according to the present invention in which said socket has a depth exceeding 1 mm and said inner surface is provided with said surface composition. As will be realized the effect of microbial corrosion in such cavities may be reduced by utilizing the teachings of the invention. It is a further advantage that the inner surface is provided with a thin uniform surface composition which is enabled by the invention. Especially, when there is at least one thread or other precise shapes for enabling perfect fit between components. A thin uniform surface composition helps in keeping the precision still providing resistance for microbial effects.

An alternative embodiment discloses a dental implant system, in which a portion of the implant and/or unit that can be placed against the soft tissue comprises a threadless outer surface.

In accordance with yet a preferred embodiment a dental implant system is enabled, in which a portion of the implant surface composition is provided with a gradient, in which the roughness value (Sa value) of said portion is increasing from a lower value up to 0,3 µm, in the apical direction. The roughness can increase in increments or in a linier manner as well as non-linear and individually adapted if desired. In addition the portion of the implant that can be placed against the bone tissue comprises a second surface composition having a roughness in the range of 0,4-5 µm and pore diameters in the range of 0,1-10 µm, preferably 1-7 µm, which could also be provided with a gradient where roughness increases in the apical direction.

It is also accomplished a method for producing an implant and/or a spacer belonging to said implant, characterized in that it is an anodic oxidation method in which a portion of inner or outer surface/s is/are applied in a vessel comprising an electrolyte, for example comprising sulphuric acid and phosphoric acid, and the voltage (U) is below 100 Volts and more than 30 Volts and the dwell time of the portion or portions in the electrolyte are chosen such that a surface composition, largely or completely assuming the crystalline anatase phase, is formed.

Preferably, said surface composition forms part of a soft tissue facing surface of a component of an implant system. More preferably, at at least 90% of the pores have an orifice with a mean inside diagonal distance of less than 0,1 µm. Even more preferably at least 95% of the pores, preferably at least 99% of the pores have an orifice with a mean inside diagonal distance of less than 0,2 µm.

Advantageously, the surface composition has a mean roughness value (Sa value) of below 0,3µm. It is realized that the surface composition may be accomplished so as to form part of a bore or other inner surface of a component of a dental implant system.

More in detail major content of said surface composition is formed by titanium oxide in the anatase phase. Preferably, at least35 % by volume or even more preferred 50% by volume (a major content) of said surface composition is formed by titanium oxide in the anatase phase. The implant is made of titanium dioxide or an alloy thereof. At the same time it is important to maintain the thin structure of the surface composition.

The implant is formed by at least one of the options selected from a group comprising milling, turning, etching or an additive manufacturing technique, such as 3D printing, molding or stereo lithography prior to anodic oxidation. After the anodic oxidation, if desired, the implant may be provided with a coating, preferably comprising an antimicrobial agent, being provided on said surface composition in a subsequent step.

The application of titanium dioxide in the anatase phase has significantilly reduced bacteria growth in the area concerned. The titanium dioxide in the anatase phase combined with the machined surface topography can thus be used to effectively avoid bacteria absorption and growth in the area with the soft tissue exposure and in this way to avoid peri-implant infection. This guarantees a good implant survival prognosis in the long term.Some embodiments provide for a medical procedure of implanting a medical implant that is more securely and less complicated to perform for a surgeon. Some embodiments provide for increased surgical flexibility. Some embodiments provide for improved surgical control, e.g. by improved visible feedback of the medical procedure. Some embodiments provide for cost-effectiveness, e.g. by reduced surgery time, patient recovery time, potential side effects, etc.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the invention are capable of will be apparent and elucidated from the following description of embodiments of the present invention, reference being made to the accompanying drawings, in which
Figs. 1A and 1B are side views of various embodiments of dental implant systems and a spacer provided with a surface according to the invention.
Fig. 2 shows a diagrammatic side view of titanium dioxide in the anatase phase being applied to an implant by means of anodic oxidation,
Fig. 3 shows a diagrammatic side view of titanium dioxide in the anatase phase being applied to a unit or soft tissue through-piece belonging to the implant,
Fig. 4A shows, in graph form, the applied current as a function of time, and
Fig. 4B shows how the voltage is related to during the anodization process, as shown in Fig. 4B.

### Description of embodiments

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

The feature which can be regarded as characterizing an arrangement according to the invention is that the implant structure will consist of crystalline titanium dioxide which largely or completely assumes the anatase phase and that the surface structure in the micrometer range obtained by machining is preserved. In one embodiment, a large part or all of the outer surface or outer surfaces of the implant or of the spacer sleeve is provided with the crystalline titanium oxide assuming the anatase phase. However, in the preferred embodiment, the implant structure according to the invention is applied to an abutment. and the most coronal parts of the implant. The anatase layer combined with the smooth preserved structures from machining will prevent or significantly reduce microbial colonization of the surface while allowing for good integration with the surrounding tissue. The sleeve surfaces and the surfaces of the most coronal part of the dental implant according to the invention are described as follows: The sleeve and the coronal part of the implant are machined, e.g. turned, from cp titanium or Ti6Al4V alloy. The surfaces according to the invention are anodized using a method described below. They will be compared with unanodized control surfaces in the following. The surface structure was analysed by SEM and interferrometry (WYKO). The surface morphology was examined and documented using a Zeiss Ultra 55 scanning electron microscopy (SEM). Images of the surface were taken using 10kV accelerating voltage at 500x, 2000x and 8000x magnifications. The surface structure on all anodized and control components had characteristic grooves from the turning tool in the micrometer range. The anodized surfaces had a nanometer structure in the 50 nm range superimposed on the turned structure. The nanometer structure was covering the entire surface on the anodized cp titanium components, whereas only limited areas of nanometer structure were observed on the anodized alloy components.

Disks from cp titanium and titanium alloy were made by turning, and the surface roughness was measured. The mean roughness (Sa) of the surface was measured in three 630x470µm areas on all samples. The measurements were performed using a WYKO NT9100 profilometry system and data were evaluated in software Vision 4.10. Before calculating Sa data were processed by extrapolation of "dead pixels", tilt correction and smoothed by a 5x5 median filter. There was no significant difference in surface roughness for the control and anodized surfaces. The surface mean roughness Sa was 0.18, 0.18, 0.17 and 0.20 µm, respectively, for the machined cp titanium and alloy controls and for anodized cp titanium and alloy, respectively. Thus, the underlying machined surface structure was preserved in the micrometer range.

Near Edge X-ray Absorption Fine Structure (NEXAFS) experiments were performed using the synchrotron source at Max-IV Laboratory in Lund, Sweden, for assessment of surface crystallinity. The spectra indicated that the anodized surface oxides were partially crystalline with anatase as dominating phase, whereas structures typical for anatase were significantly less pronounced in spectra taken from the control surfaces. The Ti cp peak maximum for the anodised surfaces along with the shoulder on the high energy side is indicative of an anatase crystal phase on these surfaces. The observed differences in the O1s spectra were also compatible with a higher anatase content on the anodically oxidised surfaces.

Further embodiments of the novel implant and spacer surfaces are set out in the attached dependent claims concerning the implant. Following is a report on an experiment showing the effect that the new surface according to the invention has on microbial colonization.

Fresh clinical isolates of *Streptococcus gordonii* (HC7), *Streptococcus mitis* (BA7) and *Streptococcus sanguinis* (FC2) were used in this study. *Streptococcus gordonii, S. mitis* and *S*. *sanguinis* were obtained from approximal dental plaque. *Streptococcus gordonii* was identified by positive phenotypic tests for *N*-acetylglucosaminidase, *N*-acetylgalactosaminidase, α-fucosidase and β-galactosidase. Identification of *Streptococcus mitis* was based on positive phenotypic tests for *N*-acetylgalactosaminidase, *N*-acetylglucosaminidase, β-galactosidase and sialidase. while S. *sanguinis* was identified based on genotypic tests negative for sialidase, arbinosidase, L-fucosidase, α-glucosidase and firm adherence to MSA agar.

### Culture conditions

Bacteria were grown on blood agar in an atmosphere of 5 % CO₂ in air at 37 °C. Colonies were transferred to Bacto Todd-Hewitt broth (TH) (Becton Dickinson & Co) and grown overnight in 5 % CO₂ in air at 37 °C. The suspension was transferred to fresh TH broth and incubated at 37 °C until the mid-exponential growth phase was reached (OD₆₀₀ₙₘ ≈ 0.5). Cultures were then centrifuged (4000 ***g***) for 10 min and the pellets were re-suspended in the same volume of TH broth diluted 1:10 in sterile PBS before use. Suspensions of the bacteria were either used alone or mixed in equal volumes to give a four-species consortium.

### Preparation of saliva

Since the dental components become exposed to saliva in the oral cavity, in the experiment we have compared the adherence of three early colonizing oral bacterial species to the unanodized control and the two surfaces generated by anodic oxidation in the presence of a saliva coating.
Bacteria-free saliva with proteins kept in their native configuration was prepared as described by Wickström & Svensäter (2008). After ethical approval had been obtained from the Faculty of Odontology, whole saliva was collected on ice over 1 h from ten healthy volunteers. The saliva was then pooled and mixed with an equal volume of PBS, stirred gently overnight at 4 °C and centrifuged in a Beckman Coulter Avanti J-E centrifuge (20 min, 30 000 ***g*,** 4 °C). The supernatant was subjected to isopycnic density-gradient centrifugation in CsCI/0.1 M NaCl in a Beckman Coulter Optima LE-80K Ultracentrifuge at 36,000 rpm for 90 h at 15 °C. Bacteria-free fractions were collected from the top of the tube and pooled before being dialysed against PBS and stored at - 20 °C.

### Saliva pellicle

Prior to the experiments, anodized cp titanium and alloy titanium discs and unanodized control disks cp titanium were coated overnight in 12-well culture dishes with 25 % saliva in PBS at room temperature. Prior to use, unbound salivary proteins were removed from the discs by transferring to new wells filled with 3 ml PBS and incubation on a rocking platform (VWR international LLC) operating at 10 cycles min⁻¹ for 2 x 10 min.

### Adhesion assay

For the adhesion assay, saliva coated discs were placed in wells containing 3ml of bacterial suspension containing 10⁷ cells per ml and maintained on the rocking platform for 2 h at 37 °C. The discs were then washed with Todd-Hewitt broth diluted 1:10 in PBS (2 x 10 min) to remove loosely attached cells and stained with Live/Dead BacLight (Molecular Probes). Adhered cells were visualised using a fluorescence microscope.

### Image analysis and statistics

Experiments were carried out three times using independent bacterial cultures. For all experiments, ten randomly selected areas were photographed were taken avoiding the centre and extreme edges of the discs, and surface coverage was determined using the Biolmage-L image analysis software (Chavez de Paz, 2009). Differences in bacterial coverage were analysed ANOVA and P-values below 0.05 were considered significant.

### Results

### Effect of nano-structured titanium surfaces on bacterial adherence

For *S*. *gordonii, S. mitis* and *S*. *sanguinis,* the fluorescence microscopy images of the anodized cp Ti and alloy surfaces (N1 and N2) revealed a more sparse distribution of bacterial cells compared to the control surfaces. No obvious differences were seen between the two anodized surfaces.

Image analysis revealed that binding of *S*. *gordonii* to both the anodised cp Ti and alloy surfaces was lower than to the control (41 ± 5 % and 25 ± 9 % of control levels respectively). These reductions in adherence were significant at the 5% and 1% levels, respectively.
Although adherence to the alloy surface was slightly lower than that to cp Ti, the difference in level of adherence was not significant at the 5 % level. Binding of *S*. *mitis* to the anodized surfaces was also lower than to the control surface (anodized cp Ti: 33 ± 4 % of control and anodized alloy: 23 ± 2 % of control) and these reductions were significant at the 1 % level. As seen for *S*. *gordonii,* there was no significant difference in binding between the anodized surfaces. For *S*. *sanguinis,* image analysis revealed the adherence to both the anodized cp Ti and alloy surfaces to be significantly lower (p < 0.001) than to the control surface (15 ± 2 % of control and 28 ± 2 % of control, respectively). Unlike the other streptococcal species, S. *sanguinis* showed a significantly lower level of binding (p < 0.05) to the anodized cp Ti as compared to the alloy surface.

These data showed that *S*. *gordonii* and *S*. *mitis* adherence to the anodized surfaces was significantly reduced compared to control and *S*. *sanguinis* showed markedly lower adherence to the two anodized surfaces. In this study, early biofilm formation on two anodized titanium surfaces was compared with a commercially pure titanium control surface. Since salivary proteins are well known to affect the binding of bacteria to oral surfaces, (Nikawa et al. 2006; Lima et al. 2008; Mei et al. 2011) the experiments were performed in the presence of a salivary pellicle in order to model the *in vivo* situation. The purified salivary preparation used, was previously shown to contain large salivary mucins (MUC5B and MUC7) as well as a range of proteins including gp340, lysozyme, lactoferrin, α-amylase, slgA, statherin, cystatins and prolactin-inducible protein (Dorkhan et al. 2013).
For the three of the tested species, the level of binding to the anodized surfaces was less than 50% of that to the commercially pure titanium. For *S*. *gordonii* and *S*. *mitis,* this reduction was significant at the 5% and 1% level, respectively, and for *S*. *sanguinis* the reduction was significant for the 1 % level. Despite the fact that the anodized surfaces were made from of commercially pure titanium and titanium alloy respectively, both surfaces showed similar patterns of reduction in bacterial adherence compared to control.

Overall, the results of this study lead to the conclusion that the salivary pellicle formed on the anodized surfaces is different to that on the control and that this results in significantly reduced adherence of early colonizing streptococcal colonizers, especially *S*. *sanguinis* to these surfaces. This finding thus emphasises the importance of using saliva-coated surfaces in experiments to investigate bacterial colonization of dental implant materials.

Extrapolation of these data to the *in vivo* situation indicates that development of a new generation of titanium dental implants and spacer sleeves incorporating such anodized surfaces with preserved structure in the micrometer range obtained by machining could make a significant contribution to reducing the early stages of microbial biofilm formation at these sites. This could in turn reduce binding of later colonizers and the formation of complex, mature biofilms. It may be expected that a reduction in the overall bacterial load on the spacer sleeve surfaces and most coronal parts of the implants could tip the balance in favour of soft-tissue adhesion, allowing an improvement in the soft-tissue barrier at the sites. Thereby, the risk of peri-implant infection and implant treatment complication and failure is reduced.

The following is a description of the novel method used for manufacturing the new surfaces according to the invention: The feature which can principally be regarded as characterizing the novel method is that it comprises an anodic oxidation procedure providing a titanium oxide surface enriched with anatase and allowing for preserved underlying surface structure in the micrometer range resulting from machining. In this method, the part or parts bearing said outer layer or outer layers are applied to a liquid or electrolyte under voltage, e. g. sulphuric acid and phosphoric acid. The electrolyte composition and the voltage, current and the dwell time of the actual part or parts of the implant in the liquid are chosen so that titanium dioxide, partially assuming the anatase phase, is formed while preserving the underlying structure in the micrometer range. Different electrolyte compositions are associated with different voltages.
In one embodiment, the voltage is chosen with values between 30 and 99 volts. At lower voltages, the titanium dioxide layer has too low anatase content, and at higher voltages the micrometer structure of the titanium dioxide layer is transformed to include a large number of pores in the micrometer range and the characteristic structures created during machining of the substrate body are lost.

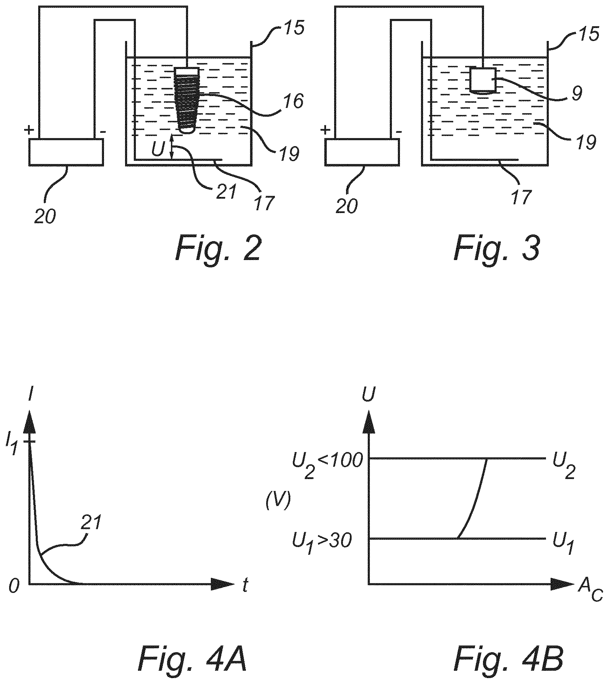

By means of what has been proposed above, an excellent and effective reduction of microbial colonization is achieved. The layer or layers also provide the possibility of effective soft tissue integration at the part or portion that can be placed against or extend through the soft tissue. The implant production is highly advantageous because methods and procedures already known per se can be used. No modifications are needed to the actual implant or unit structure, and they can be distributed and handled in the manner already practised in the dental field. Likewise, the actual implantation work can follow already established routines, with the difference that microbial growth on the components surfaces is reduced significantly. Layers with different properties of titanium dioxide in the anatase phase may be applied to an implant by means of anodic oxidation. Figures 2 and 3 show a diagrammatic side view of titanium dioxide in the anatase phase being applied to an implant and a unit or soft tissue through-piece belonging to the implant.

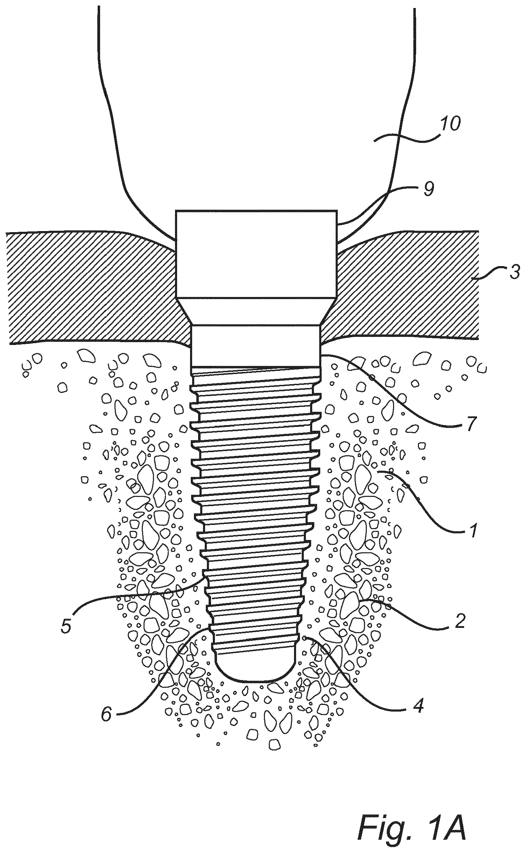

According to Figure 1A, an implant system 1 is provided in bone 2, e.g. a jaw bone of a patient. On top of the jaw bone there is a bed of soft tissue 3. The bone 2 is initially provided with a hole, shown symbolically by reference number 4. A fixture 5 which can be of a type known per se is arranged in the hole. The fixture 5 can thus comprise, for example, an outer thread 5a by means of which said fixture 5 can be screwed into the hole 4. The hole 4 can be threaded or unthreaded. The fixture 5 is also provided with a flange-like portion 7 which has a peripheral surface that can be placed against the soft tissue. The implant system 1 can also comprise or be connected to a unit or soft tissue through-piece 9, which can consist of or function as a spacer sleeve 9. On the soft tissue through-piece 9, the fixture 5 is intended to support a prosthesis, which is not shown here in any detail. The implant system shown in fig. 1A is manufactured and sold by Nobel Biocare® under the name NobelReplace®. As the skilled person is well aware of the prosthesis (10) can also be made to be connectable to a plurality of fixtures. It is then often referred to as a bridge or a bar. The bridge can be fixture supported or spacer supported or even partly supported by any of the two together with a tooth or teeth. Moreover, the prosthesis can also be made like a single restoration and be either fixture supported or connected to a spacer or abutment. All the above alternatives are well known and described in the art and not explained in further detail here. The important finding is that at least areas of any implant system that is facing soft tissue or the oral cavity or is in contact with soft tissue is preferably equipped with a surface composition according to the present invention.

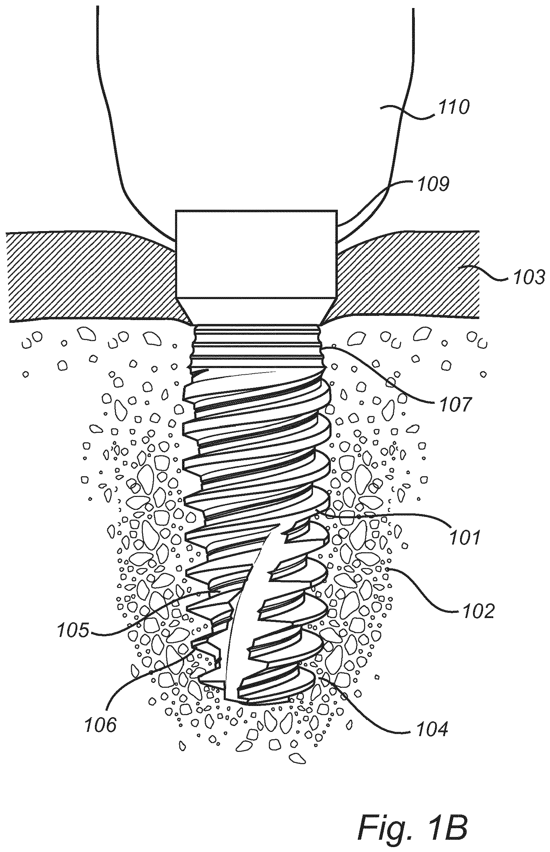

The implant according to Figure 1A can now be provided with a machined surface with structures in the micrometer range and along all or most of its outer surface with a thin layer of titanium dioxide which completely or partially, assumes the crystalline form anatase. In the case according to Figure 1A, the placement of the implant has resulted in exposure of its upper parts to soft tissue. Said anatase has been shown to have a powerful effect on reducing microbial growth and colonization and a stimulating effect on tissue integration, which in Figure 1 has been illustrated by soft tissue growth 3 surrounding the coronal part of the fixture 5 and the spacer sleeve 9 surface. The anatase structure has thus made the surface of the coronal part of the implant and spacer sleeve able to resist microbial colonization and guide soft tissue formation. The topography of the structure is following the underlying structure in the micrometer range given to the implant surface when it was produced using milling or turning as production method.

The application of titanium dioxide in the anatase phase has significantilly reduced bacteria growth in the area concerned. The titanium dioxide in the anatase phase combined with the machined surface topography can thus be used to effectively avoid bacteria absorption and growth in the area with the soft tissue exposure and in this way to avoid peri-implant infection. This allows for a good implant survival prognosis in the long term. Of course, the implant system 101 according to Figure 1B can be provided with titanium dioxide along the full outer extent of the implant. The bone hole formation is indicated by 104.

It is not disclosed in detail in the drawings a dental implant system (1), in which said socket has a depth exceeding 1 mm and said inner surface is provided with said surface composition. However, the skilled person is familiar with the design of implants and a drawing of said inner surface is not necessary here. It is realized that the inner surface is provided with a uniform surface composition layer and comprises at least one thread. The method according to the invention allows for the formation of anatase in a thin surface composition that allows for even microstructure patterns to shine through the thin surface composition.

The implant surface composition may be provided with a gradient, in which the roughness of the surface composition is increasing, within the range of a lower value up to 0,3 µm, in the apical direction. This feature can be applied to various embodiments by using various gradients. Especially it should be noted that the implant (5) that can be placed against the bone tissue (2) comprises a second surface composition having a roughness in the range of 0,4-5 µm and pore diameters in the range of 0,1-10 µm, preferably 1-7 µm. Moreover, the second surface composition is provided with a gradient, in which the roughness value is increasing, within said range, in the apical direction.

Figures 2 and 3 show the principle of anodic oxidation, in which use is made of a vessel 15 with a liquid containing an electrolyte 19, e. g. sulphuric acid and phosphoric acid. The mode of operation shall be with potentiostatic control. The process time shall be less than 10 seconds. In an anode and cathode arrangement, the implant represents an anode 16, and a contact unit a cathode 17. The implant is completely or partially immersed in the electrolyte 19. The anode and the cathode are connected respectively to the plus pole and minus pole of a voltage source which is symbolized by 20. The voltage source 20 can comprise control members of a known type to ensure that the voltage between the anode/implant and the cathode/contact unit located in the electrolyte can be varied if necessary. Thus, the voltage U can, for a certain composition of the electrolyte, be varied or set to a first value in the range of below 100 Volts and above 30 Volts. If the electrolyte has another composition, the value is set to another value which can be in the stated range, i. e. between 30 and 100 volts, so as to obtain on the outer surface or outer surfaces in question a titanium dioxide layer according to the above, which assumes the crystalline anatase phase while preserving the underlying surface structure in the micrometer range. It will be appreciated that the titanium dioxide layer can be varied in terms of thickness and phase by controlling the voltage value by means of said control members or setting members and by moving the implant in the directions of the arrows 21. The immersion time of the surface or surface parts is also crucial in determining the structure of the titanium dioxide layer.

Figure 3 shows the case where a soft tissue through-piece 9, spacer or unit 9 is coated completely or partially with titanium dioxide in the anatase phase, using equipment according to Figure 2. In the present example, the entire through-piece is immersed in the liquid bath or electrolyte 19.

Figure 4A shows how the anatase content can vary as a function of the applied current I for a certain immersion time and for a given electrolyte. The dependence of the anatase content on, inter alia, the current has been represented by the curve 21. Fig. 4B indicates a first threshold value U1 where the anatase phase occurs. As seen from Fig 4B the voltage is held constant and/or near a value U1 throughout the process.

The implant 1 and/or the soft tissue through-piece 9 thus have a portion or portions that can be placed against the jaw bone and/or soft tissue. Each such portion can be unthreaded or can be provided with a thread, groove or pattern. Different layers can be provided on locally distinct sites or on top of one another. It can also be exposed to higher voltage in order to roughen the bone tissue facing portions somewhat more. The implant can be held in different positions or continuously moved up from the electrolyte to enable formation of a gradient. The formation of a gradient can be controlled in many different ways.

## Claims

1. An implant structure or parts thereof having a surface composition **characterized in that**
said surface composition comprising titanium oxide in the anatase crystalline phase and that said surface composition has a porous structure where at least 90% of the pores have an orifice with a mean inside diagonal distance of less than 0,1 µm.

2. An implant structure according to Claim 1, in which said surface composition is obtained through an anodic oxidation process.

3. An implant structure according to any one of claims 1-2, in which said surface composition has a mean roughness value (Sa value) of below 0,3 µm.

4. An implant structure according to any one of the preceding claims, in which at least 95% of the pores, preferably at least 99% of the pores have an orifice with a mean inside diagonal distance of less than 0,2 µm.

5. An implant structure according to any one of claims 1-4,
in which said surface composition forms part of a tissue facing surface of a component of an implant system and/or
in which said surface composition forms part of a bore or other inner surface of a component of an implant system.

6. An implant structure according to any one of claims 1-5,
in which at least 35% by volume of said surface composition is formed by titanium oxide in the anatase phase or
in which a major content of said surface composition volume is formed by titanium oxide in the anatase phase.

7. An implant structure according to any one of claims 1-6,
in which said surface composition comprises phosphorus and/or
in which a coating, preferably said coating comprises an antimicrobial agent, is provided on said surface composition.

8. An implant system provided with at least one component, preferably an abutment for use in the oral cavity, comprising an implant structure according to any one of claims 1-7.

9. An implant system according to claim 8, in which said implant system is a dental implant system (1) comprising a body having a proximal end, a distal end, an outer surface extending between the proximal end and the distal end, the body having a longitudinal axis; and
a. an open socket formed in a top portion of the body, the open socket comprising an inner surface extending from the distal end towards the proximal end of the body along the longitudinal axis of the body; and
b. a spacer (9), e. g. a spacer sleeve, belonging to said dental implant system, which is/are intended to extend from said distal end in a hole (4) formed in jaw bone (2) and in soft tissue (3).

10. A dental implant system (1) according to claim 9,
in which said spacer (9) is at least one of the components of the group comprising an abutment, an overdenture bar and a fixture supported bridge and/or
in which said socket has a depth exceeding 1 mm and said inner surface is provided with said surface composition and/or
in which said inner surface is provided with a thin uniform surface composition and comprises at least one thread and/or
in which a portion of the implant (7) and/or unit (9) that can be placed against the soft tissue (3) comprises a threadless outer surface and/or
in which a portion of the implant surface composition is provided with a gradient, wherein the roughness value (Sa value) of said portion is increasing from a lower value up to 0,3 µm, in the apical direction and/or
in which said implant (7) and/or spacer (9) comprising titanium or an alloy thereof.

11. A dental implant system (1) according to any one of claims 9-10, in which a portion of the implant (5) that can be placed against the bone tissue (2) comprises a second surface composition having a roughness value in the range of 0,4-5 µm and pore diameters in the range of 0,1-10 µm, preferably 1-7 µm and preferably
in which a portion of the second surface composition is provided with a gradient, wherein the roughness value is increasing, within the range of 0,4-5 µm, in the apical direction.

12. A dental implant system (1) according to any one claims, in which a portion of the second surface composition is provided with a gradient, wherein the roughness value is increasing, within the range of 0,4-5 µm, in the apical direction.

13. A method for producing an implant and/or a spacer belonging to said implant according to any one of claims 1 to 7, **characterized in that** it is an anodic oxidation method in which a portion of inner or outer surface/s is/are applied in a vessel (15) comprising an electrolyte (19), for example comprising sulphuric acid and phosphoric acid, and the voltage (U) is below 100 Volts and more than 30 Volts and the dwell time of the portion or portions in the electrolyte (19) are chosen such that a surface composition, largely or completely assuming the crystalline anatase phase, is formed.

14. The method according to claim 13,
in which a coating, preferably comprising an antimicrobial agent, being provided on said surface composition in a subsequent step and/or
in which said implant is formed by at least one of the options selected from a group comprising milling, turning, etching or an additive manufacturing technique, such as 3D printing, molding or stereo lithography prior to anodic oxidation.

15. The method according to claim 13 or 14,
in which the voltage (U) is chosen with values between 30 and 99 Volts and/or
in which the electrolyte (19) comprises sulphuric acid and phosphoric acid and/or
in which a process time is less than 10 seconds.

## Patentansprüche

1. Implantatstruktur oder Teile davon, die eine Oberflächenzusammensetzung aufweist, **dadurch gekennzeichnet, dass**
die Oberflächenzusammensetzung Titanoxid in der Anataskristallphase umfasst und dadurch, dass die Oberflächenzusammensetzung eine poröse Struktur aufweist, wobei mindestens 90 % der Poren eine Öffnung mit einem mittleren Innendiagonalabstand von weniger als 0,1 µm aufweisen.

2. Implantatstruktur nach Anspruch 1, in welcher die Oberflächenzusammensetzung durch einen anodischen Oxidationsprozess gewonnen wird.

3. Implantatstruktur nach einem der Ansprüche 1-2, in welcher die Oberflächenzusammensetzung einen mittleren Rauheitswert (Sa-Wert) von unter 0,3 µm aufweist.

4. Implantatstruktur nach einem der vorhergehenden Ansprüche, in welcher mindestens 95 % der Poren, vorzugsweise mindestens 99 % der Poren, eine Öffnung mit einem mittleren Innendiagonalabstand von weniger als 0,2 µm aufweisen.

5. Implantatstruktur nach einem der Ansprüche 1-4,
in welcher die Oberflächenzusammensetzung einen Teil einer einem Gewebe zugewandten Oberfläche einer Komponente eines Implantatsystems bildet, und/oder
in welcher die Oberflächenzusammensetzung einen Teil einer Bohrung oder einer anderen inneren Oberfläche einer Komponente eines Implantatsystems bildet.

6. Implantatstruktur nach einem der Ansprüche 1-5,
in welcher mindestens 35 Volumen-% der Oberflächenzusammensetzung aus Titanoxid in der Anatasphase gebildet sind, oder
in welcher ein Hauptteil des Volumens der Oberflächenzusammensetzung durch Titanoxid in der Anatasphase gebildet ist.

7. Implantatstruktur nach einem der Ansprüche 1-6,
in welcher die Oberflächenzusammensetzung Phosphor umfasst, und/oder
in welcher eine Beschichtung, wobei die Beschichtung vorzugsweise ein antimikrobielles Mittel umfasst, auf der Oberflächenzusammensetzung bereitgestellt ist.

8. Implantatsystem, das mit mindestens einer Komponente, vorzugsweise einem Anschlag zur Verwendung im Mundraum versehen ist, die eine Implantatstruktur nach einem der Ansprüche 1-7 umfasst.

9. Implantatsystem nach Anspruch 8, in welchem das Implantatsystem ein Dentalimplantatsystem (1) ist, das einen Körper umfasst, der ein proximales Ende, ein distales Ende, eine Außenoberfläche, die sich zwischen dem proximalen Ende und dem distalen Ende erstreckt aufweist, wobei der Körper eine Längsachse aufweist; und
a. einen offenen Sockel, der auf einem oberen Abschnitt des Körpers ausgebildet ist, wobei der offene Sockel eine innere Oberfläche umfasst, die sich von dem distalen Ende in Richtung des proximalen Endes des Körpers entlang der Längsachse des Körpers erstreckt; und
b. einen Abstandhalter (9), z. B. eine Abstandhülse, der zu dem Dentalimplantatsystem gehört, welche(r) dazu bestimmt ist/sind, sich von dem distalen Ende in ein Loch (4) zu erstrecken, das im Kieferknochen (2) oder in einem Weichgewebe (3) ausgebildet ist.

10. Dentalimplantatsystem (1) nach Anspruch 9,
in welchem der Abstandhalter (9) mindestens eine der Komponenten aus der Gruppe bestehend aus einem Anschlag, einer Deckprothesenstange und einer durch eine Befestigung getragenen Brücke ist, und/oder
in welchem der Sockel eine Tiefe aufweist, die 1 mm überschreitet, und die innere Oberfläche mit der Oberflächenzusammensetzung versehen ist, und/oder in welchem die innere Oberfläche mit einer dünnen einheitlichen Oberflächenzusammensetzung versehen ist und mindestens ein Gewinde umfasst, und/oder
in welchem ein Abschnitt des Implantats (7) und/oder einer Einheit (9), der gegen das weiche Gewebe (3) platziert werden kann, eine gewindelose Außenoberfläche umfasst, und/oder in welchem ein Abschnitt der Implantatoberflächenzusammensetzung mit einem Gradienten versehen ist, wobei der Rauheitswert (Sa-Wert) des Abschnitts sich von einem niedrigeren Wert auf bis zu 0,3 µm in die apikale Richtung erhöht, und/oder
in welchem das Implantat (7) und/oder der Abstandhalter (9) Titan oder eine Legierung davon umfassen.

11. Dentalimplantatsystem (1) nach einem der Ansprüche 9-10, in welchem ein Abschnitt des Implantats (5), der gegen das Knochengewebe (2) platziert werden kann, eine zweite Oberflächenzusammensetzung umfasst, die einen Rauheitswert in dem Bereich von 0,4-5 µm und Porendurmesser in dem Bereich von 0,1-10 µm, vorzugsweise 1-7 µm, aufweist, und vorzugsweise in welchem ein Abschnitt der zweiten Oberflächenzusammensetzung mit einem Gradienten versehen ist, wobei der Rauheitswert sich innerhalb des Bereichs von 0,4-5 µm in die apikale Richtung erhöht.

12. Dentalimplantatsystem (1) nach einem der Ansprüche, in welchem ein Abschnitt der zweiten Oberflächenzusammensetzung mit einem Gradienten versehen ist, wobei sich der Rauheitswert in dem Bereich von 0,4-5 µm in die apikale Richtung erhöht.

13. Verfahren zum Produzieren eines Implantats und/oder eines Abstandhalters, der zu dem Implantat nach einem der Ansprüche 1 bis 7 gehört, **dadurch gekennzeichnet, dass** es ein anodisches Oxidationsverfahren ist, in welchem ein Abschnitt von (einer) inneren/äußeren Oberfläche(n) in ein Gefäß (15) eingebracht ist, das ein Elektrolyt (19), zum Beispiel umfassend Schwefelsäure und Phosphorsäure, umfasst, und wobei die Spannung (U) unterhalb von 100 Volt und bei mehr als 30 Volt liegt und die Haltezeit des Abschnitts oder der Abschnitte in dem Elektrolyt (19) derartig ausgesucht ist, dass eine Oberflächenzusammensetzung, die größtenteils oder vollkommen in der Kristallanatasphase vorliegt, gebildet wird.

14. Verfahren nach Anspruch 13,
in welchem eine Beschichtung, vorzugsweise umfassend ein antimikrobielles Mittel, das auf der Oberflächenzusammensetzung in einem nachfolgenden Schritt bereitgestellt wird, und/oder in welchem das Implantat durch mindestens eine Option ausgewählt aus einer Gruppe bestehend aus Fräsen, Drehen, Ätzen oder einer additiven Herstellungstechnik, wie 3D-Druck, Formen oder Stereolithografie vor der anodischen Oxidation gebildet wird.

15. Verfahren nach Anspruch 13 oder 14,
in welchem die Spannung (U) mit Werten zwischen 30 und 99 Volt ausgesucht ist, und/oder
in welchem das Elektrolyt (19) Schwefelsäure und Phosphorsäure umfasst, und/oder
in welchem eine Prozesszeit weniger als 10 Sekunden beträgt.

## Revendications

1. Structure d'implant ou parties de celle-ci ayant une composition de surface **caractérisée en ce que**
ladite composition de surface comprenant un oxyde de titane dans la phase cristalline anatase et **en ce que** ladite composition de surface a une structure poreuse où au moins 90 % des pores ont un orifice avec une distance diagonale interne moyenne de moins de 0,1 µm.

2. Structure d'implant selon la revendication 1, dans laquelle ladite composition de surface est obtenue par l'intermédiaire d'un procédé d'oxydation anodique.

3. Structure d'implant selon l'une quelconque des revendications 1 et 2, dans laquelle ladite composition de surface a une valeur de rugosité moyenne (valeur Sa) inférieure à 0,3 µm.

4. Structure d'implant selon l'une quelconque des revendications précédentes, dans laquelle au moins 95 % des pores, de préférence au moins 99 % des pores ont un orifice avec une distance diagonale interne moyenne de moins de 0,2 µm.

5. Structure d'implant selon l'une quelconque des revendications 1 à 4,
dans laquelle ladite composition de surface forme une partie d'une surface orientée vers un tissu d'un composant d'un système d'implant et/ou
dans laquelle ladite composition de surface forme une partie d'un alésage ou d'une autre surface interne d'un composant d'un système d'implant.

6. Structure d'implant selon l'une quelconque des revendications 1 à 5,
dans laquelle au moins 35 % en volume de ladite composition de surface sont formés par un oxyde de titane dans la phase anatase ou
dans laquelle une teneur majeure dudit volume de composition de surface est formée par un oxyde de titane dans la phase anatase.

7. Structure d'implant selon l'une quelconque des revendications 1 à 6,
dans laquelle ladite composition de surface comprend du phosphore et/ou
dans laquelle un revêtement, de préférence ledit revêtement comprend un agent antimicrobien, est fourni sur ledit composition de surface.

8. Système d'implant pourvu d'au moins un composant, de préférence une butée pour une utilisation dans la cavité buccale, comprenant une structure d'implant selon l'une quelconque des revendications 1 à 7.

9. Système d'implant selon la revendication 8, dans lequel ledit système d'implant est un système d'implant dentaire (1) comprenant un corps ayant une extrémité proximale, une extrémité distale, une surface externe s'étendant entre l'extrémité proximale et l'extrémité distale, le corps ayant un axe longitudinal ; et
a. une douille ouverte formée dans un portion supérieure du corps, la douille ouverte comprenant une surface interne s'étendant à partir de l'extrémité distale vers l'extrémité proximale du corps le long de l'axe longitudinal du corps ; et
b. une espaceur (9), par exemple un manchon d'espacement, appartenant audit système d'implant dentaire, qui est/sont destiné(s) à s'étendre à partir de ladite extrémité distale dans un trou (4) formé dans un os de mâchoire (2) et dans un tissu mou (3).

10. Système d'implant dentaire (1) selon la revendication 9, dans lequel ledit espaceur (9) est au moins l'un des composants du groupe comprenant une butée, une barre de prothèse supradentaire et un bridge supporté par une fixation et/ou dans lequel ladite douille a une profondeur supérieure à 1 mm et ladite surface interne est pourvue de ladite composition de surface et/ou
dans lequel ladite surface interne est pourvue d'une composition de surface mince et uniforme et comprend au moins un filetage et/ou
dans lequel une portion de l'implant (7) et/ou de l'unité (9) qui peut être placée contre le tissu mou (3) comprend une surface externe sans filetage et/ou
dans lequel une portion de la composition de surface d'implant est pourvue d'un gradient, dans lequel la valeur de rugosité (valeur Sa) de ladite portion augmente à partir d'une valeur inférieure jusqu'à 0,3 µm, dans la direction apicale et/ou dans lequel ledit implant (7) et/ou ledit espaceur (9) comprenant du titane ou un alliage de celui-ci.

11. Système d'implant dentaire (1) selon l'une quelconque des revendications 9 et 10, dans lequel une portion de l'implant (5) qui peut être placée contre le tissu osseux (2) comprend une seconde composition de surface ayant une valeur de rugosité dans la plage allant de 0,4 à 5 µm et des diamètres de pore dans la plage allant de 0,1 et 10 µm, de préférence de 1 à 7 µm et de préférence
dans lequel une portion de la seconde composition de surface est pourvue d'un gradient, dans lequel la valeur de rugosité augmente, dans la plage allant de 0,4 à 5 µm, dans la direction apicale.

12. Système d'implant dentaire (1) selon l'une quelconque des revendications, dans lequel une portion de la seconde composition de surface est pourvue d'un gradient, dans lequel la valeur de rugosité augmente, dans la plage allant de 0,4 à 5 µm, dans la direction apicale.

13. Procédé pour produire un implant et/ou un espaceur appartenant audit implant selon l'une quelconque des revendications 1 à 7, **caractérisé en ce que** c'est un procédé d'oxydation anodique dans lequel une portion des surfaces interne ou externe est appliquée dans un récipient (15) comprenant un électrolyte (19), par exemple comprenant de l'acide sulfurique et de l'acide phosphorique, et la tension (U) est inférieure à 100 volts et supérieure à 30 volts et le temps de séjour de la portion ou des portions dans l'électrolyte (19) est choisi de telle sorte qu'une composition de surface, prenant en grande partie ou totalement la phase cristalline anatase, est formée.

14. Procédé selon la revendication 13,
dans lequel un revêtement, comprenant de préférence un agent antimicrobien, est fourni sur ledit composition de surface dans une étape ultérieure et/ou
dans lequel ledit implant est formé par au moins l'une des options sélectionnées dans un groupe comprenant le fraisage, le tournage, la gravure ou une technique de fabrication additive, telle que l'impression 3D, le moulage ou la stéréolithographie avant l'oxydation anodique.

15. Procédé selon la revendication 13 ou 14,
dans lequel la tenson (U) est choisie avec des valeurs entre 30 et 99 volts et/ou
dans lequel l'électrolyte (19) comprend de l'acide sulfurique et de l'acide phosphorique et/ou
dans lequel une durée du procédé est inférieure à 10 secondes.
